(19) Europäisches Patentamt
European Patent Office
Office européen des brevets

(11) Veröffentlichungsnummer : **0 318 567 B1**

# (12) EUROPÄISCHE PATENTSCHRIFT

(45) Veröffentlichungstag der Patentschrift : **18.03.92 Patentblatt 92/12**

(51) Int. Cl.$^5$ : **C07D 319/12**

(21) Anmeldenummer : **88905758.4**

(22) Anmeldetag : **15.06.88**

(86) Internationale Anmeldenummer : **PCT/EP88/00531**

(87) Internationale Veröffentlichungsnummer : **WO 88/10260 29.12.88 Gazette 88/28**

## (54) MESO-LACTID UND VERFAHREN ZU SEINER HERSTELLUNG.

Die Akte enthält technische Angaben, die nach dem Eingang der Anmeldung eingereicht wurden und die nicht in dieser Patentschrift enthalten sind.

(30) Priorität : **16.06.87 DE 3720060**

(43) Veröffentlichungstag der Anmeldung : **07.06.89 Patentblatt 89/23**

(45) Bekanntmachung des Hinweises auf die Patenterteilung : **18.03.92 Patentblatt 92/12**

(84) Benannte Vertragsstaaten : **AT BE CH DE FR GB IT LI LU NL SE**

(56) Entgegenhaltungen :
**EP-A- 0 261 572**
**DE-A- 2 118 127**
**FR-A- 1 478 694**
**Chemical Abstracts, Band 105, 1986, (Columbus, Ohio, US), D.R. Cowsar et al.: "Poly(lactide-co-glycolide) microcapsules for controlled release of steroids", siehe Seite 329, Zusammenfassung 102505x, & Methods Enzymol. 1985, 112(Drug Enzyme Targeting, Pt. A.), 101-16**
**Chemical Abstracts, Band 106, Nr. 14, 6. April 1987, (Columbus, Ohio, US), A.K. Khomyakov et al.: "Cationic copolymerization of glycolide with optically active and racemic lactide under the action of tin dichloride dihydrate", siehe Seiten 1, 2, Zusammenfassung 102705h, & Vysokomol. Soedin., Ser.A 1986, 28 (10), 2217-22**
**Römpps Chemie-Lexikon, 8. Auflage, Seite 2305**
**J. of Dairy Science 43 (1960), Seiten 1421-1429**

(73) Patentinhaber : **BOEHRINGER INGELHEIM KG**
**Postfach 200**
**W-6507 Ingelheim am Rhein (DE)**
(84) **AT BE CH DE FR GB IT LI LU NL SE**

Patentinhaber : **BOEHRINGER INGELHEIM INTERNATIONAL GmbH**
**Postfach 20**
**W-6507 Ingelheim am Rhein (DE)**
(84) **GB**

(72) Erfinder : **MÜLLER, Manfred**
**Alsbacher Str. 40**
**W-6101 Bickenbach (DE)**
Erfinder : **HESS, Joachim**
**Mozartstr. 8**
**W-6530 Bingen (DE)**
Erfinder : **SCHNELL, Wilhelm-Gustav**
**Brüder-Grimm-Str. 1A**
**W-6507 Ingelheim (DE)**
Erfinder : **BENDIX, Dieter**
**Veith-Stoss-Str. 17**
**W-6507 Ingelheim (DE)**
Erfinder : **ENTENMANN, Günther**
**Schützenpfad 16**
**W-6507 Ingelheim (DE)**

**EP 0 318 567 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft meso-Lactid, Verfahren zu seiner Herstellung und seine Verwendung zur Herstellung von Polymeren und Copolymeren.

Polymere auf der Basis von Milchsäure besitzen ein vorrangiges Interesse beim Einsatz in der Chirurgie und in der Wundversorgung, da diese Polymere im Körper zu natürlichen Stoffwechselprodukten abgebaut werden. Die Einsatzmöglichkeiten von biologisch abbaubaren Polymeren und Copolymeren sind aus dem Stand der Technik hinreichend bekannt und brauchen im einzelnen nicht näher erläutert werden. So können z.B. im Bereich der Osteosynthese durch den Einsatz abbaubarer Polymere die bislang bei der Verwendung von Metallimplantaten notwendigen Zweitoperationen zur Entfernung des Implantats vermieden werden. Seitdem bekannt ist, daß die Zusammensetzung und die Herstellung (Polymerisationsbedingungen, Polymerisationsgeschwindigkeit) der Polymeren einen wesentlichen Einfluß auf die Stabilität (Zug- und Biegefestigkeit) und die Abbaurate der Polymere hat, besteht ein großes Interesse an optisch reinen, wohldefinierten Monomeren. Im Fall der Lactide werden als "Monomere" die entsprechenden dimeren cyclischen Ester eingesetzt.

Während von L(-)-, D(+) und racemischem D,L-Lactid die "reinen" Verbindungen bekannt sind, ist das meso-Lactid in reiner Form bislang unbekannt.

In der Literatur ist die Definition von D,L-Lactid und meso-Lactid nicht immer konsequent, im Sinne einer korrekten Nomenklatur, eingehalten.

Im Sinne der Erfindung wird als meso-Lactid der cyclische Diester der Milchsäure I bezeichnet,

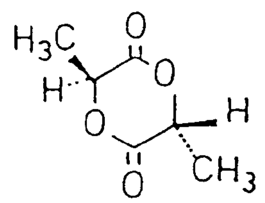

I

der zwei Asymmetriezentren mit entgegengesetzter Konfiguration R und S aufweist.

Zwar wird in der DE-A 2 118 127 ein meso-Lactid mit einem Schmelzpunkt von 124 bis 125°C beschrieben, jedoch liegt bei dieser Angabe offensichtlich eine Verwechslung vor, da dieser Schmelzpunkt üblicherweise D,L-Lactid zugeordnet wird [Römpps Chemie-Lexikon, 8. Auflage, Franck'sche Verlagsbuchhandlung Stuttgart, 1983, Seite 2305)], während demgegenüber für meso-Lactid im Stand der Technik ein Schmelzpunkt im Bereich von 42 bis 45°C angegeben wird [D.D. Deane und E.G. Hammond, Journal of Dairy Science 43 (1960) 1421].

Es ist die Aufgabe der vorliegenden Erfindung ein "reines" meso-Lactid zur Verfügung zu stellen, das als Monomer zur Herstellung von biologisch abbaubaren Polymeren und Copolymeren eingesetzt werden kann.

Die Aufgabe wird erfindungsgemäß dadurch gelöst, daß ein Gemisch aus D,L-Lactid und meso-Lactid unter vermindertem Druck rektifiziert wird.

Aus dem ersten Destillat erhält man nach dem Umkristallisieren ein reines meso-Lactid vom Schmelzpunkt 52.8°C.

Gegenstand der Erfindung ist somit ein meso-Lactid, das einen Schmelzpunkt von ca. 52 - vorzugsweise 52.8°C - aufweist.

Ein weiterer Gegenstand der vorliegenden Anmeldung sind Verfahren zur Herstellung des erfindungsgemäßen meso-Lactids sowie das jeweils nach diesen Verfahren hergestelle meso-Lactid. - Diese betreffen ein Verfahren zur Herstellung von meso-Lactid, welches darin besteht, daß man ein Gemisch aus D,L-Lactid und meso-Lactid unter vermindertem Druck mittels einer Kolonne, die eine theoretische Bodenzahl von mindestens 30 aufweist, rektifiziert und gegebenenfalls das resultierende meso-Laktid aus einem $C_1$- bis $C_4$-Alkohol oder Toluol umkristallisiert sowie ein weiteres Verfahren zur Herstellung von meso-Lactid, welches darin besteht, daß man racemische Polymilchsäure in Gegenwart von 0,05 bis 1,00 Gew.-% Zinnstaub, $Zinn^{2+}$-Salzen oder in Gegenwart einer organischen Zinnverbindung unter vermindertem Druck auf eine Temperatur im Bereich von 130°C bis 230°C erhitzt und das sich bildende Lactid abdestilliert, aus einem $C_1$- bis $C_4$-Alkohol fällt, das resultierende Kristallisat aus einem $C_1$- bis $C_4$-Alkohol umkristallisiert und das so erhaltenen Kristallisat in einem halogenierten Kohlenwasserstoff oder in einem aliphatischen Ether löst, das D,L-Laktid auskristallisieren

läßt, die Mutterlauge einengt, unter vermindertem Druck mit einer Kolonne, die eine theoretische Bodenzahl von mindestens 30 aufweist rektififziert und gegebenenfalls das daraus resultierende meso-Laktid aus einem $C_1$- bis $C_4$-Alkohol oder aus Toluol umkrisallisiert.

Ein weiterer Gegenstand der Erfindung ist die Verwendung des erfindungsgemäß hergestellten meso-Lactids zur Herstellung von Polymeren und Copolymen des folgenden Struktur:

a) Polymesolactide hergestellt durch Polymerisation des erfindungsgemäßen meso-Lactids;

b) resorbierbare Copolymerisate, Einheiten von Polymesolactid enthaltend, welche ihrerseits aus dem erfindungsgemäßen meso-Lactid hergestellt worden sind - und

c) Copolymerisate hergestellt durch Polymerisation des erfindungsgemäßen meso-Lactids und mindestens einem Monomer ausgewählt aus der Gruppe der 1,3-Dioxan-2-one der Struktur II, 1,4-Dioxan-2-one der Struktur III, Lactide der Struktur IV und Lactone der Struktur V,

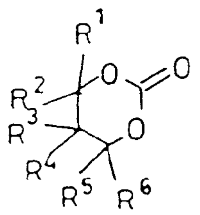

II

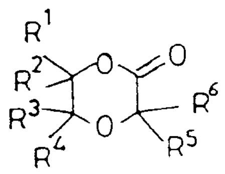

III

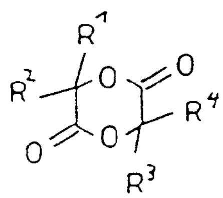

IV

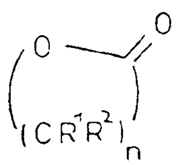

V

worin $R^1$, $R^2$, $R^3$, $R^4$, $R^5$, $R^6$ gleich oder verschieden sein und Wasserstoff, eine verzweigte oder unverzweigte Alkyl-, Alkenyl- oder Alkinylgruppe mit 1 bis 12, bevorzugt 1 bis 4 Kohlenstoffatomen, die gegebenenfalls durch Halogen, bevorzugt Chlor, Brom oder Jod, Hydroxy, eine verzweigte oder unverzweigte Alkoxygruppe mit 1 bis 4 Kohlenstoffatomen, eine Formylgruppe; eine Acylgruppe mit 1 bis 5 Kohlenstoffatomen, eine Carboxylgruppe, eine Aminogruppe, eine Alkylamino-, Dialkylamino- oder quartäre Aminogruppe mit bevorzugt 1 bis 4 Kohlstoffatomen substituiert sein kann, eine Cycloalkylgruppe mit 3 bis 6 Kohlenstoffatomen, die wie eine Alkylgruppe substituiert sein kann, einen Fromylrest, eine Acylgruppe mit 1 bis 5 Kohlenstoffatomen, eine verzweigte oder unverzweigte Alkoxycarbonylgruppe mit 1 bis 5 Kohlenstoffatomen in der Alkoxygruppe, eine gegebenenfalls substituierte Aryl- oder Hetarylgruppe mit 6 bis 12 Kohlenstoffatomen im Ringsystem und n eine der Zahlen 2, 3, 4, 5, 6, 7, 8, 9 oder 10 bedeuten.

Ein weiterer Gegenstand der vorliegenden Erfindung ist ein Copolymerisat auf der Basis des erfindungsgemäßen meso-Lactids und mindestens einem Monomer ausgewählt aus der Gruppe L-Lactid, D-Lactid, Glycolid, Trimethylencarbonat und/oder 1,4-Dioxanon insbesondere ein poly(meso-lactid-Co-glycolid).

Ein weiterer Gegenstand der vorliegenden Erfindung ist die Verwendung eines der erfindungsgemäßen polymerisate oder Copolymerisate zur Herstellung von chirurgischen und medizischen Materialen - insbesondere die Verwendung

– in einem verstreckten und orientierten Filament, welches allein oder im Gemisch mit Filamenten aus anderen polymeren zur Herstellung von chirurgischem Nahtmaterial verwendet wird;

– in einem verstreckten und orientierten Filament, welches allein oder im Gemisch mit Filamenten aus anderen Polymeren als Verstärkungsmaterial in einem chirurgischen Gegenstand enthalten ist;

– in einer pharmazeutischen Zubereitung, die einen oder mehrere Arzneistoffe über einen bestimmten Zeitraum geregelt abgibt;

– in chirurgischen Gegenständen, Knochenersatz- oder Knochenverbundmaterialien und anderen endoprothetischen Gegenständen.

Aus der Literatur ist bislang lediglich ein meso-Lactid mit einem Schmelzpunkt von 41 bis 45°C bekannt.

Das Ausgangsmaterial wird wie folgt hergestellt: Racemische Polymilchsäure (50:50/D:L) wird in Gegenwart eines geeigneten Katalysators, z.B. in Gegenwart von 0.05 bis 1.0 Gew.-% Zinnstaub, $Sn^{2+}$-Salzen oder einer organischen Zinnverbindung, abgeleitet von einer Carbonsäure mit bis zu 20 Kohlenstoffatomen bei vermindertem Druck auf 130 bis 230°C erhitzt, das sich bildende Lactid abdestilliert und kontinuierlich oder batchweise Polymilchsäure nachgefüllt.

Als Katalysatoren besonders geeignete Zinnverbindungen sind Verbindungen der allgemeinen Struktur

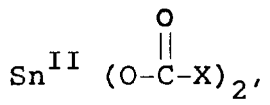

worin X einen verzweigten oder unverzweigten Alkyl- oder Alkenylrest mit bis zu 19 Kohlenstoffatomen oder einen Naphthylrest bedeutet, oder Verbindungen der allgemeinen Struktur

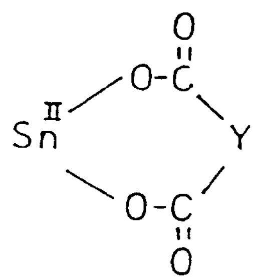

worin Y einen verzweigten oder unverzweigten Alkylen- oder Alkenylenrest mit bis zu 18 Kohlenstoffatomen oder einen Phenylrest bedeutet.

Bevorzugte Katalysatoren sind Zinntartrat, Zinnoxalat, Zinndicaprylat, Zinndilaurat, Zinndipalmitat, Zinndistearat, das Zinndioleat (Derivat der Ölsäure), Zinn-α-naphthoeat oder Zinn-β-naphthoeat. Besonders bevorzugt ist Zinndioctoat, besser bezeichnet als Zinn-di-(2-ethylhexanoat), oder Zinnstaub.

Die in das Verfahren eingesetzte racemische Polymilchsäure kann in einem gesonderten Reaktionsschritt nach bekannten Verfahren durch Entwässern von racemischer Milchsäure hergestellt werden.

In einer anderen Ausführungsform des Verfahrens wird anstelle der Polymilchsäure Milchsäure eingesetzt. In einem ersten Reaktionsschritt wird die Milchsäure in Gegenwart des Katalysators bei vermindertem Druck bei ansteigender Temperatur entwässert. Im allgemeinen erfolgt die Entwässerung bei Drucken von ca. 10 bis 50 hPa, wobei die Temperatur im Reaktor auf ca. 150 bis 170°C ansteigt. Wenn ein durchschnittliches Molekulargewicht der dabei gebildeten Polymilchsäure von ca. 400 bis 2000, bevorzugt 600 - 800, erreicht ist, wird kontinuierlich Lactid abdestilliert und anschließend kontinuierlich oder batchweise Polymilchsäure nachgefüllt.

In einer weiteren Ausführungsform kann in das kontinuierliche Verfahren anstelle von Polymilchsäure auch Milchsäure nachgezogen werden.

Beim Anfahren des Reaktors wird racemische Polymilchsäure vorgelegt und mit 0.05 bis 1.0 Gew.-%, bevorzugt 0.1 bis 0.8 Gew.-% Zinnstaub oder der zinnorganischen Verbindung versetzt. Anschließend wird bei vermindertem Druck auf 130 bis 230°C, bevorzugt 180 bis 200°C erhitzt, wobei das entstehende rohe Lactid, d.h. ein Gemisch aus D,L-Lactid und meso-Lactid abdestilliert wird. Der optimale Temperaturbereich ist von dem angelegten Vakuum abhängig und kann durch einfache Versuche ermittelt werden. Eine möglichst niedrige Destillationstemperatur wirkt sich günstig auf die Reinheit des Destillats aus. Nachdem eine bestimmte Produktmenge abdestilliert ist, wird racemische Polymilchsäure nachgefüllt. Dies geschieht zweckmäßigerweise in geschmolzener Form. Das Nachfüllen kann batchweise (portionsweise) oder aber auch in kontinuierlicher Form, z.B. durch Zupumpen, geschehen. Dabei kann die nachgefüllte Menge durchaus größer sein als die ursprüngliche, beim Start der Reaktion eingesetzte Menge.

Für den Fall, daß die racemische Polymilchsäure batchweise nachgefüllt wird, ist das Restvolumen des Reaktorinhalts in einem weiten Bereich bezüglich der Qualität des Produktes unkritisch, es ist jedoch zweckmäßig, nach ca. 50 bis 90%igem Umsatz aufzufüllen.

Es ist nicht auszuschließen, daß ein zu weites Absenken des Reaktorinhalts zu einer Produktverschlechterung führt. Im Fall der kontinuierlichen Produktführung erfolgt der Zulauf zweckmäßig so, daß das Volumen des Reaktorinhalts weitestgehend konstant gehalten wird.

Wird anstelle von racemischer Polymilchsäure direkt racemische Milchsäure in den Reaktor eingesetzt, erfolgt vor der Depolymerisation zu dem rohen Lactid in Gegenwart des Katalysators der zinnorganischen Verbindung oder des Zinnstaubs eine Entwässerung der Milchsäure zu Polymilchsäure bis zu einem mittleren Molekulargewicht von ca. 400 - 2000, bevorzugt 500 bis 800. Die Entwässerung erfolgt vorzugsweise bei ca.

30 hPa bei ansteigender Temperatur bis ca. 170°C. Nachdem das gewünschte Molekulargewicht erreicht ist, wird der Ansatz wie zuvor beschrieben weiterverarbeitet.

Wie bereits ausgeführt, kann in einer weiteren Verfahrensvariante anstelle von racemischer Polymilchsäure auch racemische Milchsäure nachgezogen werden, wobei dann zuerst wieder Wasser aus dem Reaktionsgemisch bis zum Erhalt des gewünschten Molekulargewichtes der Polymilchsäure abdestilliert wird. Das weitere Verfahren erfolgt dann wie oben beschrieben.

Der Einsatz bzw. das Nachfüllen von Milchsäure anstelle von Polymilchsäure hat gegenüber den zuvor beschriebenen Varianten keinerlei Nachteile bezüglich der Ausbeute. Von Vorteil ist, daß die Reaktionszeit zur Entwässerung der Milchsäure um ca. 50 % verkürzt wird. Das Molekulargewicht der entstehenden Polymilchsäure wird durch Titration der Endgruppen bestimmt.

Das abdestillierte rohe Lactid, das aus einem Gemisch von meso- und D,L-Lactid besteht, wird anschließend zuerst aus einem $C_1$- bis $C_4$-Alkohol (wie z.B. Methanol, Ethanol, n-Propanol, Isopropanol), bevorzugt Isopropanol, gefällt. Das daraus erhaltene Kristallisat, das aus einem Gemisch von meso- und D,L-Lactid besteht, wird dann zuerst aus einem $C_1$- bis $C_4$-Alkohol (wie z.B. Methanol, Ethanol, n-Propanol, Isopropanol), bevorzugt Isopropanol umkristallisiert. Dieses so erhaltene zweite Kristallisat wird dann in einem halogenierten Kohlenwasserstoff, bevorzugt Chloroform, besonders bevorzugt 1,2-Dichlorethan, oder einem aliphatischen Ether, bevorzugt Diethylether, gelöst, wobei anschließend D,L-Lactid auskristallisiert wird. Die verbleibende Mutterlauge, bestehend aus einem Gemisch von meso-Lactid und D,L-Lactid, im Verhältnis von ca. 60 : 40, wird eingeengt und der Rückstand zur weiteren Trennung erfindungsgemäß rektifiziert. Hierbei wird aus dem ersten Destillat das erfindungsgemäBe meso-Lactid erhalten. Gegebenenfalls kann dies zur weiteren Reinigung umkristallisiert werden. Bevorzugte Lösungsmittel sind $C_1$ - $C_4$-Alkohole, bevorzugt Isopropanol, oder Toluol.
Diese Umkristallisation dient in erster Linie dazu, die bei der Destillation in geringem Maß gebildete freie Säure zu entfernen.

Es ist selbstverständlich, daß vor dem gewünschten ersten Destillat aus meso-Lactid ein Vorlauf verworfen wird. Der Vorlauf besteht im allgemeinen aus Lösungsmittelresten, die aus dem Eindampfen der Mutterlauge herrühren und eventuell aus geringfügigen Mengen Reaktionsprodukten aus dem Lösungsmittel und dem Lactid, wie z.B. der Isopropylester, wenn Isopropanol als Lösungsmittel benutzt wurde. Die Rektifikation kann mit dem Stand der Technik entsprechenden Destillationskolonnen, die eine bestimmte Mindesttrennleistung aufweisen (Bodenzahl, variables Rücklaufverhältnis) durchgeführt werden. Einetheoretische Mindestbodenzahl, die zur Trennung zweier oder mehrerer Komponenten erforderlich ist, ergibt sich allgemein aus der Siedepunktsdifferenz der zu trennenden Komponenten.

Wie aus den Diagrammen der Siedepunktskurven (Abb. I und II) ersichtlich, unterscheiden sich die Siedepunkte von reinem meso-Lactid und D,L-Lactid in einem weiten Druckbereich um ca. 7°C. Hieraus ergibt sich, daß eine destillative Trennung der beiden Lactide mit Destillationskolonnen möglich ist, die eine theoretische Mindestbodenzahl von ca. 30 - 40 aufweisen. Der Zusammenhang zwischen der theoretischen Bodenzahl und der tatsächlichen Trennleistung, der praktischen Bodenzahl, einer Kolonne hängt von vielen Faktoren ab, kann jedoch durch einfache Versuche bestimmt werden. Wird diese Mindestbodenzahl von der verwendeten Apparatur nicht erreicht, so ist eine mehrmalige Destillation erforderlich.

Das nach dem erfindungsgemäßen Verfahren hergestellte meso-Lactid wird durch die folgenden physikalischen Parameter charakterisiert.

Schmelzpunkt 52.77°C bestimmt nach dem DTA-Verfahren
(Differential-Thermo-Analyse) (Abb. IIa)
GC: 99.8 % meso-Lactid, 0,2 % D,L-Lactid
(Gaschromatographische Trennung)
$[\alpha]_D^{20} = 0°$
IR (gemessen in KBr und als Film in Nujol)
Abb. IIIa und IIIb
$^1$H-NMR (400 Megaherz) siehe Abb. IVa und IVb

Bislang ist ein meso-Lactid dieser Reinheit nicht bekannt und wird als solches beansprucht.

Das erfindungsgemäße meso-Lactid ist aufgrund seiner optischen Reinheit besonders zur Herstellung definierter Polymere und Copolymere geeignet.

Im Sinne der Erfindung betrifft dies insbesondere ein Poly(meso-lactid), hergestellt aus dem erfindungsgemäßen "reinen" Monomer wie auch ein Copolymerisat, hergestellt durch Polymerisation von meso-Lactid unter Verwendung nachfolgend genannter Monomere.

Geeignet hierfür sind 1,3-Dioxan-2-one der Struktur II, 1,4-Dioxan-2-one der Struktur III, Lactide der Struktur IV und Lactone der Stuktur V

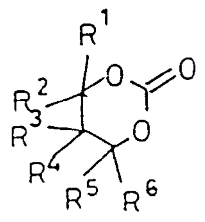

II

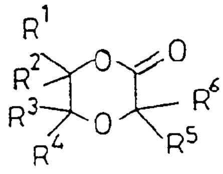

III

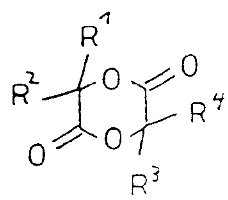

IV

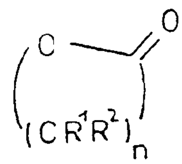

V

In den Strukturformeln II bis V können $R^1$, $R^2$, $R^3$, $R^4$, $R^5$, $R^6$ gleich oder verschieden sein und Wasserstoff, eine verzweigte oder unverzweigte Alkyl-, Alkenyl- oder Alkinylgruppe mit 1 bis 12, bevorzugt 1 bis 4 Kohlenstoffatomen, die gegebenenfalls durch Halogen, bevorzugt Chlor, Brom oder Jod, Hydrory, eine verzweigte oder unverzweigte Alkorygruppe mit 1 bis 4 Kohlenstoffatomen, eine Formylgruppe; eine Acylgruppe mit 1 bis 5 Kohlenstoffatomen, eine Carborylgruppe, eine Aminogruppe, eine Alkylamino-, Dialkylamino- oder quartäre Aminogruppe mit bevorzugt 1 bis 4 Kohlenstoffatomen substituiert sein kann, eine Cycloalkylgruppe mit 3 bis 6 Kohlenstoffatomen, die wie eine Alkylgruppe substituiert sein kann, einen Formylrest, eine Acylgruppe mit 1 bis 5 Kohlenstoffatomen, eine verzweigte oder unverzweigte Alkoxycarbonylgruppe mit 1 bis 5 Kohlenstoffatomen in der Alkoxygruppe, eine gegebenenfalls substituierte Aryl- oder Hetarylgruppe mit 6 bis 12 Kohlenstoffatomen im Ringsystem und n eine der Zahlen 2, 3, 4, 5, 6, 7, 8, 9 oder 10 bedeuten. Unter Halogen wird ein Atom aus der Gruppe Fluor, Chlor, Brom oder Jod verstanden, bevorzugte Alkylgruppen sind, soweit nichts anderes angegeben, Methyl, Ethyl, Propyl, iso-Propyl, Butyl, tert.- Butyl, bevorzugte Arylgruppen sind Phenyl oder substituiertes Phenyl. Es ist vorausgesetzt, daß Reste an gleichen C-Atomen so auszuwählen sind, daß die Herstellung der Verbindungen der allgemeinen Formeln II bis V aufgrund des Standes der Technik möglich ist.

Bevorzugte Monomere sind:
L-Lactid, D-Lactid, D,L-Lactide, Glycolid,
Trimethylencarbonat und 1,4-Dioxanon.

Das Verhältnis der Monomeren kann in weiten Bereichen zwischen 1 bis 99 zu 99 bis 1 Mol % (meso-Lactid: Monomer) gewählt werden.

Bevorzugte Copolymerisate sind:
Poly(meso-lactid-co-L-lactid,
Poly(meso-lactid-co-D-lactid),
Poly(meso-lactid-co-D,L-lactid)
Poly(meso-lactid-co-glycolid)
Poly(meso-lactid-co-trimethylencarbonat)

Im Sinne der Erfindung werden nicht nur "Random"-Polymerisate, sondern auch Block-Polymerisate beansprucht.

Im Gegensatz zu bekannten Polymesolactiden, - die aus Monomeren erhalten werden, die einen Schmelzpunkt von ca. 45°C aufweisen, - weist das erfindungsgemäße Polymesolactid eine rein syndiotaktische Struktur auf.

Die erfindungsgemäß hergestellten Polymerisate können beispielsweise zur Herstellung von Nahtmaterialien, Wirkstoffträgern, chirurgischen Implantaten - Platten, Schrauben und Stiften zum Fixieren von Knochenelementen - Prothesen und Füllmaterialien - Netzen und Folien zur Weichteilrekonstruktion - Folien, geschäumten Folien und Pudern zur Wundbehandlung verwendet werden.

Die erfindungsgemäß beanspruchten Polymere und Copolymere können in Analogie zu bekannten Verfahren aus den beschriebenen Monomeren hergestellt werden. Hierzu gehören Verfahren, die in der Schmelze, in Lösung oder in Suspension durchgeführt werden.

Geeignete Polymerisationskatalysatoren sind beispielsweise organische Zinn-Verbindungen - bevorzugt Zinn-(II)-Octoat

[Zinn-di-(2-ethylhexanoat)] und Zinn-Salze, wie z.B. Zinn-(II)-chlorid. Im allgemeinen beträgt die Reaktionsdauer der Polymerisation zwischen einer und 72 Stunden; die Reaktionstemperatur zwischen 100 und 180°C.

Im nachfolgenden wird die Erfindung anhand von Beispielen näher erläutert.

Beispiele für die Herstellung von meso-Lactid

Beispiel 1:

920 kg rac.-Milchsäure mit einem Gehalt von ca. 90% werden bis ca 180°C Sumpftemperatur und ca. 10 hPa entwässert. Man erhält 700 kg rac. Polymilchsäure mit einem durchschnittlichen Molekulargewicht von 600.

Beispiel 2:

430 kg rac. Polymilchsäure mit einem durchschnittlichen Molekulargewicht von 600 werden mit 3,5 kg mit dem Zinn-(II)-Salz der 2-Ethylhexansäure [Zinndioctoat] versetzt und auf 194°C-198°C, bei einem Vacuum von 33-17 hPa, erhitzt. Hierbei destilliert D,L-Lactid und meso-Lactid ab. Nach einer Destillatmenge von 374 kg Lactid werden 270 kg der oben beschriebenen Polymilchsäure nachgezogen und unter vorgenannten Bedingungen 252 kg Lactid erhalten.

Rückstand: 20 kg
Durchsatz: Lactid: ca 40 kg/h

Das Destillat von 626 kg wird in gleicher Menge Isopropanol gefällt und geschleudert. Man erhält 545,6 kg Lactid (feucht). Dieses wird aus 818 kg Isopropanol umkristallisiert, geschleudert und getrocknet. Man erhält 395 kg eines Gemisches aus D,L- und meso-Lactid. Dieses Gemisch wird in 198 kg 1,2-Dichlorethan umkristallisiert, geschleudert und getrocknet. Man erhält 241 kg D,L-Lactid als Kristallisat, das nochmals in 362 kg Toluol zu 219 kg (33,1% bezogen auf den Einsatz von Milchsäure) D,L-Lactid umkristallisiert wird.

| | | | |
|---|---|---|---|
| Aussehen: | weiße Kristalle | | |
| Drehung: | 0,0° | | |
| Schmp.: | DTA 124,6°C | | |
| Wasser: | 0,012% | | |
| Gehalt: | nach Verseifung: | 99,6 | % |
| | Freie Säure : | 0,001 | % |
| | GC | 0,15 | % Toluol |

Das Kristallisat enthält ausschließlich D,L-Lactid und ist praktisch frei von meso-Lactid.

Beispiel 3:

Die bei der D,L-Lactid-Umkristallisiation aus 1,2-Dichlorethan erhaltene Mutterlauge (Beispiel 2) wird bis zum Rückstand eingeengt und mit 147 kg Isopropanol versetzt. Nach dem Abdestillieren von ca.31 kg Isopropanol wird abgekühlt und der erhaltene Kristallbrei geschleudert und getrocknet. Man erhält 120,4 kg (18,2% bez. auf Einsatz Milchsäure) meso-Lactid mit einem Gehalt von ca. 63%. (Rest = D,L-Lactid)

Beispiel 4:

8000 g meso-Lactid (nach Beispiel 3) mit einem Gehalt von ca. 63% werden in einer Destillationskolonne von 1 = 1,80 m, ∅ = 40 mm, gefüllt mit Maschendrahtringen 6/6 mm bei 8 hPa und 147°C Sumpftemperatur bei einem Rücklaufverhältnis von = 15 : 1 rektifiziert. Man erhält 3985 g (75,1 % d. Th.) meso-Lactid 95%ig.

Beispiel 5

8050 g meso-Lactid (nach Beispiel 3) mit einem Gehalt von ca. 63 % werden in einer Destillationskolonne von 1 = 1,80 m, ∅ = 40 mm gefüllt, mit Maschendrahtringen 6/6 mm bei 4 hPa und 147°C Sumpftemperatur bei einem Rücklaufverhältnis von 10 : 1 rektifiziert. Man erhält 3650 g (64,8 % d. Th.) meso-Lactid 89,1%ig.

Beispiel 6:

9271 g meso-Lactid 89,1%ig werden unter oben genannten Bedingungen erneut rektifiziert. Man erhält 3923 g meso-Lactid, die aus 4000 g Isopropanol umkristallisiert werden. Nach Trocknung werden 3786 g meso-Lactid 99,6%ig aus 3860 g Toluol umkristallisiert. Nach Trocknung erhält man 2969 g (35,9 % d. Th.) meso-Lactid folgender Qualität:

| | |
|---|---|
| Aussehen: | weiße Kristalle |
| Drehung: | $[\alpha]^{20}_{D}$ 0° |
| Schmp.: | DTA 52,77°C (gemessen mit DSC 2 von Perkin Elmer im Aluminium-Pfännchen, verdeckelt mit Loch). |
| Wasser: | 0,0265% |
| freie Säure: | 0,055% |
| GC | 99.8 % meso-Lactid 0,2 % D,L-Lactid |

Beispiele zur Herstellung von Polymeren und Copolymeren aus meso-Lactid

Vergleichsbeispiel

Zwei 20 g-Proben eines meso-Lactids mit einem Schmelzpunkt von 42°C, einem Drehwert $[\alpha]^{20}_{D}$ von 0°, einem Wassergehalt von 0,06 % und einer freien Säure von 0,62 % werden unter Zusatz von 137,5 ppm Zinn in Form von Zinn-(II)-octoat als Katalysator 72 Stunden bei 105° C im Vakuum polymerisiert.

Die erhaltenen Polymerisate werden durch die Bestimmung der inhärenten Viskosität (I.V.) in Chloroform bei 25° C in einer Konzentration von 0,1 g/dl charakterisiert:

Probe 1: I.V. = 0,83 dl/g
Probe 2: I.V. = 0,88 dl/g

Beispiel 7

Zwei 20 g-Proben eines meso-Lactids mit einem Schmelzpunkt von 52,8°C (Onset im DSC), einem Drehwert $[\alpha]^{20}_{D}$ von 0°, einem Wassergehalt von 0,03 % und einer freien Säure von 0,06 % werden unter Zusatz von 550,0 ppm Zinn in Form von Zinn-(II)-octoat als Katalysator 72 Stunden bei 105°C im Vakuum polymerisiert.

Die erhaltenen Polymerisate werden durch die Bestimmung der inhärenten Viskosität in Chloroform bei 25°C in einer Konzentration von 0,1 g/dl charakterisiert:

Probe 1: I.V. = 2,59 dl/g
Probe 2: I.V. = 2,41 dl/g

Der Restmonomergehalt, bestimmt durch ein 400 MHz $^{1}$H-NMR, gemessen in Deuterochloroform, beträgt 1,9 Gew.-%.

Beispiel 8

Zwei 5 g-Proben eines meso-Lactids mit einem Schmelzpunkt von 52,8°C (Onset in DSC), einem Drehwert $[\alpha]^{20}_{D}$ von 0°, einem Wassergehalt von 0,03 % und einer freien Säure von 0,06 % werden unter Zusatz von 117,2 ppm Zinn in Form von Zinn-(II)-octoat als Katalysator 8 Stunden bei 160 °C im Vakuum polymerisiert.

Die erhaltenen Polymerisate werden durch die Bestimmung der inhärenten Viskosität in Chloroform und Dioxan bei 25°C und in Hexafluorisopropanol (HFIP) bei 30°C jeweils in einer Konzentration von 0,1 g/dl charakterisiert:

| | I.V. ($CHCl_3$) [dl/g] | (Dioxan) [dl/g] | (HFIP) [dl/g] |
|---|---|---|---|
| Probe 1 | 0,68 | 0,59 | 1,08 |
| Probe 2 | 0,70 | 0,57 | 1,03 |

Beispiel 9

Eine 1 kg-Probe eines meso-Lactis mit einem Schmelzpunkt von 52,8°C (Onset im DSC), einem Drehwert $[\alpha]_D^{20}$ von 0°, einem Wassergehalt von 0,03 % und einer freien Säure von 0,06 % wird unter Zusatz von 137,5 ppm Zinn in Form von Zinn-(II)-octoat als Katalysator 72 Stunden bei 105°C im Vakuum polymerisiert.

Der erhaltene Polymerblock wird durch die Bestimmung der inhärenten Viskosität in Chloroform bei 25°C in einer Konzentration von 0,1 g/dl charakterisiert:

I.V. = 2,61 dl/g

Der Restmonomergehalt, bestimmt durch ein 400 MHz $^1$H-NMR, gemessen in Deuterochloroform, beträgt 1,7 Gew.-%.

Das Spektrum zeigt ferner einen syndiotaktischen Aufbau des Polymeren.

Das Material besitzt eine Biegefestigkeit von > 140 N/mm$^2$.

Beispiel 10

2,77 g (50 mol %) eines meso-Lactids mit einem Schmelzpunkt von 52,8°C (Onset im DSC), einem Drehwert $[\alpha]_D^{20}$ von 0°, einem Wassergehalt von 0,03 % und einer freien Säure von 0,06 % und 2,23 g Glykolid (50 mol %) wird unter Zusatz von 117,2 ppm Zinn in Form von Zinn-(II)-octoat als Katalysator bei 160°C zwischen 1 und 6 Stunden im Vakuum polymerisiert.

Die erhaltenen Copolymere werden durch die Bestimmung der inhärenten Viskosität in Chloroform und Dioxan bei 25°C und in Hexafluorisoproanol (HFIP) bei 30°C jeweils in einer Konzentration von 0,1 g/dl charkakterisiert:

| Zeit [h] | I.V. ($CHCl_3$) [dl/g] | (Dioxan) [dl/g] | (HFIP) [dl/g] |
|---|---|---|---|
| 1 | 0,88 | 0,91 | 1,83 |
| 2 | 0,87 | 0,90 | 1,76 |
| 3 | 0.87 | 0,88 | 1,69 |
| 5 | 0,81 | 0,81 | 1,58 |
| 6 | 0,82 | 0,86 | 1,60 |

Beispiel 11

2,26 g (40 mol %) eines meso-Lactids mit einem Schmelzpunkt von 52,8°C (Onset im DSC), einem Drehwert $[\alpha]_D^{20}$ von 0°, einem Wassergehalt von 0,03 % und einer freien Säure von 0,06 % und 2,23 g Glykolid (60 mol %) werden unter Zusatz von 117,2 ppm Zinn in Form von Zinn-(II)-octoat als Katalysator bei 160°C zwischen 1 und 6 Stunden im Vakuum polymerisiert.

Die erhaltenen Copolymere wurden durch die Bestimmung der inhärenten Viskosität in Chloroform und Dioxan bei 25°C und in Hexafluorisopropanol (HFIP) bei 30°C jeweils in einer Konzentration von 0,1 g/dl charakterisiert:

| Zeit [h] | I.V. ($CHCl_3$) [dl/g] | (Dioxan) [dl/g] | (HFIP) [dl/g] |
|---|---|---|---|
| 1 | n.n. | 0,98 | 2,01 |
| 2 | n.n. | 1,02 | 2,04 |
| 3 | n.n. | 0,98 | 2,04 |
| 5 | 0,82 | 1,06 | 2,04 |
| 6 | 0,81 | 0,98 | 1,97 |

n.n. = nicht in Chloroform löslich

## Patentansprüche

1. Meso-Lactid, dadurch gekennzeichnet, daß es einen Schmelzpunkt von ca. 52°C, vorzugsweise 52,8°C, aufweist.

2. Verfahren zur Herstellung von meso-Lactid gemäß Anspruch 1, dadurch gekennzeichnet, daß man ein Gemisch aus D,L-Lactid und meso-Lactid unter vermindertem Druck mittels einer Kolonne, die eine theoretische Bodenzahl von mindestens 30 aufweist, rektifiziert und gegebenenfalls das resultierende meso-Laktid aus einem $C_1$- bis $C_4$-Alkohol oder Toluol umkristallisiert.

3. Meso-Lactid, dadurch gekennzeichnet, daß es nach dem Verfahren gemäß Anspruch 2 erhalten wird.

4. Verfahren zur Herstellung von meso-Lactid gemäß Anspruch 1, dadurch gekennzeichnet, daß man racemische Polymilchsäure in Gegenwart von 0,05 bis 1,00 Gew.-% Zinnstaub, Zinn$^{2+}$-Salzen oder in Gegenwart einer organischen Zinnverbindung unter vermindertem Druck auf eine Temperatur im Bereich von 130°C bis 230°C erhitzt und das sich bildende Lactid abdestilliert, aus einem $C_1$- bis $C_4$-Alkohol fällt, das resultierende Kristallisat aus einem $C_1$- bis $C_4$-Alkohol umkristallisiert und das so erhaltene Kristallisat in einem halogenierten Kohlenwasserstoff oder in einem aliphatischen Ether löst, das D,L-Laktid auskristallisieren läßt, die Mutterlauge einengt, unter vermindertem Druck mit einer Kolonne, die eine theoretische Bodenzahl von mindestens 30 aufweist rektifiziert und gegebenenfalls das daraus resultierende meso-Laktid aus einem $C_1$- bis $C_4$-Alkohol oder aus Toluol umkristallisiert.

5. Meso-Lactid, dadurch gekennzeichnet, daß es nach dem Verfahren gemäß Anspruch 2 oder dem Verfahren gemäß Anspruch 4 erhalten und aus dem ersten Destillat mit einem Schmelzpunkt von ca. 52°C, vorzugsweise 52,8°C, gewonnen wird.

6. Verwendung von meso-Lactid gemäß einem der vorhergehenden Ansprüche zur Herstellung von Polymeren und Copolymeren.

7. Polymesolactid, hergestellt durch Polymerisation von meso-Lactid gemäß einem der Ansprüche 1 bis 5.

8. Resorbierbares Copolymerisat, dadurch gekennzeichnet, daß es Einheiten von Polymesolactid - hergestellt aus meso-Lactid gemäß einem der Ansprüche 1 bis 5 - enthält.

9. Copolymerisat hergestellt durch Polymerisation von meso-Lactid gemäß einem der Ansprüche 1 bis 5 und mindestens einem Monomer ausgewählt aus der Gruppe der 1,3-Dioxan-2-one der Struktur II, 1,4-Dioxan-2-one der Struktur III, Lactide der Struktur IV und Lacton der Struktur V,

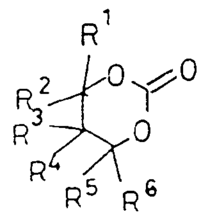 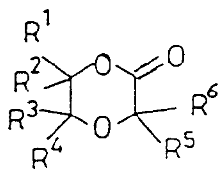 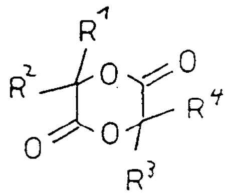

II III IV

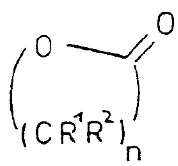

V

worin $R^1$, $R^2$, $R^3$, $R^4$, $R^5$, $R^6$ gleich oder verschieden sein und Wasserstoff, eine verzweigte oder unverzweigte Alkyl-, Alkenyl- oder Alkinylgruppe mit 1 bis 12, bevorzugt 1 bis 4 Kohlenstoffatomen, die gegebenenfalls durch Halogen, bevorzugt Chlor, Brom oder Jod, Hydroxy, eine verzweigte oder unverzweigte Alkoxygruppe mit 1 bis 4 Kohlenstoffatomen, eine Formylgruppe; eine Acylgruppe mit 1 bis 5 Kohlenstoffatomen, eine Carboxylgruppe, eine Aminogruppe, eine Alkylamino-, Dialkylamino- oder quartäre Aminogruppe mit bevorzugt 1 bis 4 Kohlenstoffatomen substituiert sein kann, eine Cycloalkylgruppe mit 3 bis 6 Kohlenstoffatomen, die wie eine Alkylgruppe substituiert sein kann, einen Formylrest, eine Acylgruppe mit 1 bis 5 Kohlenstoffatomen, eine verzweigte oder unverzweigte Alkoxycarbonylgruppe mit 1 bis 5 Kohlenstoffatomen in der Alkoxygruppe, eine gegebenenfalls substituierte Aryl- oder Hetarylgruppe mit 6 bis 12 Kohlenstoffatomen im Ringsystem und n eine der Zahlen 2, 3, 4, 5, 6, 7, 8, 9 oder 10 bedeuten .

10. Copolymerisat auf der Basis von meso-Lactid - gemäß einem der Ansprüche 1 bis 5 - und mindestens einem Monomer ausgewählt aus der Gruppe L-Lactid, D-Lactid, Glycolid, Trimethylencarbonat und/oder 1,4-Dioxanon.

11. Poly(meso-lactid-co-glycolid) gemäß Anspruch 10.

12. Verwendung eines Polymerisats oder Copolymerisats gemäß einem der Ansprüche 7 bis 11 zur Herstellung von resorbierbaren chirurgischen und medizinischen Materialien.

13. Verwendung eines Polymerisates oder Copolymerisates gemäß einem der Ansprüche 7 bis 11

– in einem verstreckten und orientierten Filament, welches allein oder im Gemisch mit Filamenten aus anderen polymeren zur Herstellung von chirurgischem Nahtmaterial verwendet wird;

– in einem verstreckten und orientierten Filament, welches allein oder im Gemisch mit Filamenten aus anderen Polymeren als Verstärkungsmaterial in einem chirurgischen Gegenstand enthalten ist;

– in einer pharmazeutischen Zubereitung, die einen oder mehrere Arzneistoffe über einen bestimmten Zeitraum geregelt abgibt;

– in chirurgischen Gegenständen, Knochenersatz- oder Knochenverbundmaterialien und anderen endoprothetischen Gegenständen.

## Claims

1. Meso-lactide, characterised in that it has a melting point of about 52°C, preferably 52.8°C.

2. Process for preparing meso-lactide according to claim 1, characterised in that a mixture of D,L-lactide and meso-lactide is rectified under reduced pressure by means of a column having a theoretical number of plates of at least 30, and optionally the resulting meso-lactide is recrystallised from a $C_{1-4}$-alcohol or toluene.

3. Meso-lactide, characterised in that it is obtained using the process according to claim 2.

4. Process for preparing meso-lactide according to claim 1, characterised in that racemic polylactic acid is heated to a temperature in the range from 130°C to 230°C in the presence of 0.05 to 1.00 wt.-% of powdered tin, $tin^{2+}$ salts or in the presence of an organic tin under reduced pressure and the lactide thus formed is distilled off, precipitated from a $C_{1-4}$-alcohol, the resulting crystals are recrystallised from a $C_{1-4}$-alcohol and the crystals thus obtained are dissolved in a halogenated hydrocarbon or in an aliphatic ether, the D,L-lactide is allowed to crystallise out, the mother liquor is concentrated by evaporation, the mixture is rectified under reduced pressure with a column having a theoretical number of plates of at least 30 and, optionally, the resulting meso-lactide

is recrystallised from a $C_{1-4}$-alcohol or from toluene.

5. Meso-lactide, characterised in that it is obtained by the process according to claim 2 or the process according to claim 4 and is recovered from the first distillate with a melting point of about 52°C, preferably 52.8°C.

6. Use of meso-lactide according to one of the preceding claims for producing polymers and copolymers.

7. Polymesolactide, prepared by polymerisation of meso-lactide according to one of claims 1 to 5.

8. Resorbable copolymer, characterised in that it contains units of polymesolactide, prepared from meso-lactide according to one of claims 1 to 5.

9. Copolymer prepared by polymerisation of meso-lactide according to one of claims 1 to 5 and at least one monomer selected from the group comprising the 1,3-dioxan-2-ones of structure II, 1,4-dioxan-2-ones of structure III, lactides of structure IV and lactones of structure V

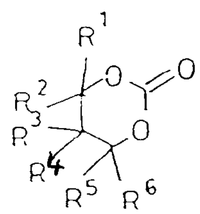

II

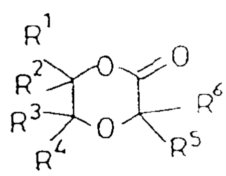

III

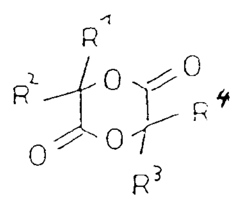

IV

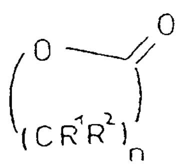

V

wherein $R^1$, $R^2$, $R^3$, $R^4$, $R^5$ and $R^6$ may be identical or different and may represent hydrogen, a branched or unbranched alkyl, alkenyl or alkynyl group having 1 to 12, preferably 1 to 4 carbon atoms, which may optionally be substituted by halogen, preferably chlorine, bromine or iodine, hydroxy, a branched or unbranched $C_{1-4}$-alkoxy group, a formyl group; a $C_{1-5}$-acyl group, a carboxyl group, an amino group or an alkylamino, dialkylamino or quaternary amino group preferably having 1 to 4 carbon atoms; a $C_{3-6}$-cycloalkyl group which may be substituted like an alkyl group, a formyl group, a $C_{1-5}$-acyl group, a branched or unbranched alkoxycarbonyl group having 1 to 5 carbon atoms in the alkoxy group, an optionally substituted aryl or hetaryl group having 6 to 12 carbon atoms in the ring system and n represents one of the numbers 2, 3, 4, 5, 6, 7, 8, 9 or 10.

10. Copolymer based on meso-lactide according to one of claims 1 to 5 and at least one monomer selected from the group comprising L-lactide, D-lactide, glycolide, trimethylenecarbonate and/or 1,4-dioxanone.

11. Poly(meso-lactide-co-glycolide) according to claim 10.

12. Use of a polymer or copolymer according to one of claims 7 to 11 for preparing resorbable surgical and medical materials.

13. Use of a polymer or copolymer according to one of claims 7 to 11

– in a stretched and oriented filament which is used on its own or mixed with filaments of other polymers to produce surgical suturing material;

– in a stretched, oriented filament which is contained on its own or mixed with filaments of other polymers as a reinforcing material in a surgical object;

– in a pharmaceutical preparation which releases one or more medicaments in controlled manner over a specific length of time;

– in surgical objects, bone replacement or bone connecting materials and other endoprosthetic objects.

## Revendications

1. Méso-lactide caractérisé en ce qu'il présente un point de fusion d'environ 52°C, de préférence de 52,8°C.

2. Procédé de préparation du méso-lactide selon la revendication 1, caractérisé en ce que l'on rectifie un mélange de D,L-lactide et de méso-lactide sous pression réduite à l'aide d'une colonne qui présente un nombre de plateaux théoriques d'au moins 30, et l'on recristallise éventuellement le méso-lactide résultant dans un

alcool en $C_1$ à $C_4$ ou dans le toluène.

3. Méso-lactide caractérisé en ce qu'il est obtenu par le procédé selon la revendication 2.

4. Procédé de préparation du méso-lactide selon la revendication 1, caractérisé en ce que l'on chauffe de l'acide polylactique racémique en présence de 0,05 à 1,00% en poids de poudre d'étain, de sels de $Sn^{2+}$ ou en présence d'un composé organique de l'étain sous pression réduite à une température située dans la gamme de 130°C à 230°C et on distille le lactide qui se forme, on le précipite dans un alcool en $C_1$ à $C_4$, on recristallise dans un alcool en $C_1$ à $C_4$ le produit cristallisé résultant et on dissout le produit cristallisé ainsi obtenu dans un hydrocarbure halogéné ou dans un éther aliphatique, on laisse cristalliser le D,L-lactide, on concentre la liqueur mère, on rectifie sous pression réduite avec une colonne qui présente un nombre de plateaux théoriques d'au moins 30 et on recristallise éventuellement le méso-lactide résultant dans un alcool en $C_1$ à $C_4$ ou dans le toluène.

5. Méso-lactide caractérisé en ce qu'il est préparé par le procédé selon la revendication 2 ou par le procédé selon la revendication 4 et en ce qu'il est obtenu à partir du premier distillat avec un point de fusion d'environ 52°C, de préférence de 52,8°C.

6. Utilisation du méso-lactide selon l'une des revendications précédentes pour la préparation de polymères et de copolymères.

7. Polymésolactide préparé par polymérisation du méso-lactide selon l'une des revendications 1 à 5.

8. Produit de copolymérisation résorbable, caractérisé en ce qu'il contient des unités de polymésolactide préparées à partir du méso-lactide selon l'une des revendications 1 à 5.

9. Produit de copolymérisation préparé par polymérisation du méso-lactide selon l'une dés revendications 1 à 5 et d'au moins un monomère choisi dans le groupe formé par les 1,3-dioxanne-2-ones de structure II, les 1,4-dioxanne-2-ones de structure III, les lactides de structure IV et les lactones de structure V

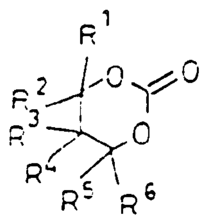

II

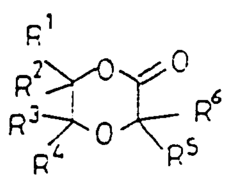

III

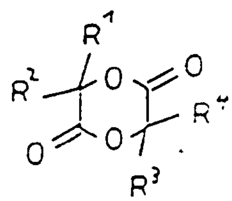

IV

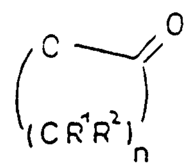

V

dans lesquels $R^1$, $R^2$, $R^3$, $R^4$, $R^5$, $R^6$ peuvent être identiques ou différents et représentent l'hydrogène, un groupe alkyle, alcényle ou alcynyle ramifié ou non ramifié de 1 à 12, de préférence de 1 à 4 atomes de carbone, qui peut être éventuellement substitué par halogène, de préférence chlore, brome ou iode, hydroxy, un groupe alcoxy ramifié ou non ramifié de 1 à 4 atomes de carbone, un groupe formyle, un groupe acyle de 1 à 5 atomes de carbone, un groupe carboxyle, un groupe amine, un groupe alkylamine, dialkylamine ou amine quaternaire ayant de préférence 1 à 4 atomes de carbone, et représentent aussi un groupe cycloalkyle de 3 à 6 atomes de carbone qui peut être substitué comme un groupe alkyle, un reste formyle, un groupe acyle de 1 à 5 atomes de carbone, un groupe alcoxycarbonyle ramifié ou non ramifié de 1 à 5 atomes de carbone dans le groupe alcoxy, un groupe aryle ou hétéroaryle éventuellement substitué de 6 à 12 atomes de carbone dans le système cyclique et n représente l'un des nombres 2, 3, 4, 5, 6, 7, 8, 9 ou 10.

10. Produit de copolymérisation à base de méso-lactide selon l'une des revendications 1 à 5 et d'au moins un monomère choisi dans le groupe du L-lactide, D-lactide, glycolide, triméthylènecarbonate et/ou 1,4-dioxanone.

11. poly(méso-lactide-co-glycolide) selon la revendication 10.

12. Utilisation d'un produit de polymérisation ou d'un produit de copolymérisation selon l'une des revendications 7 à 11 pour la préparation de produits chirurgicaux et médicaux résorbables.

13. Utilisation d'un produit de polymérisation ou d'un produit de copolymérisation selon l'une des revendications 7 à 11

– dans un filament étiré et orienté qui est utilisé seul ou en mélange avec des filaments en d'autres polymères pour la fabrication d'un produit à coudre chirurgical;
– dans un filament étiré et orienté qui est contenu seul ou en mélange avec des filaments en d'autres polymères en tant que produit de renforcement dans un article chirurgical;
– dans une préparation pharmaceutique qui libère un ou plusieurs médicaments de manière réglée en une durée déterminée;
– dans des articles chirurgicaux, des prothèses osseuses ou des matériaux composites osseux et d'autres articles d'endoprothèses.

ABB. 1

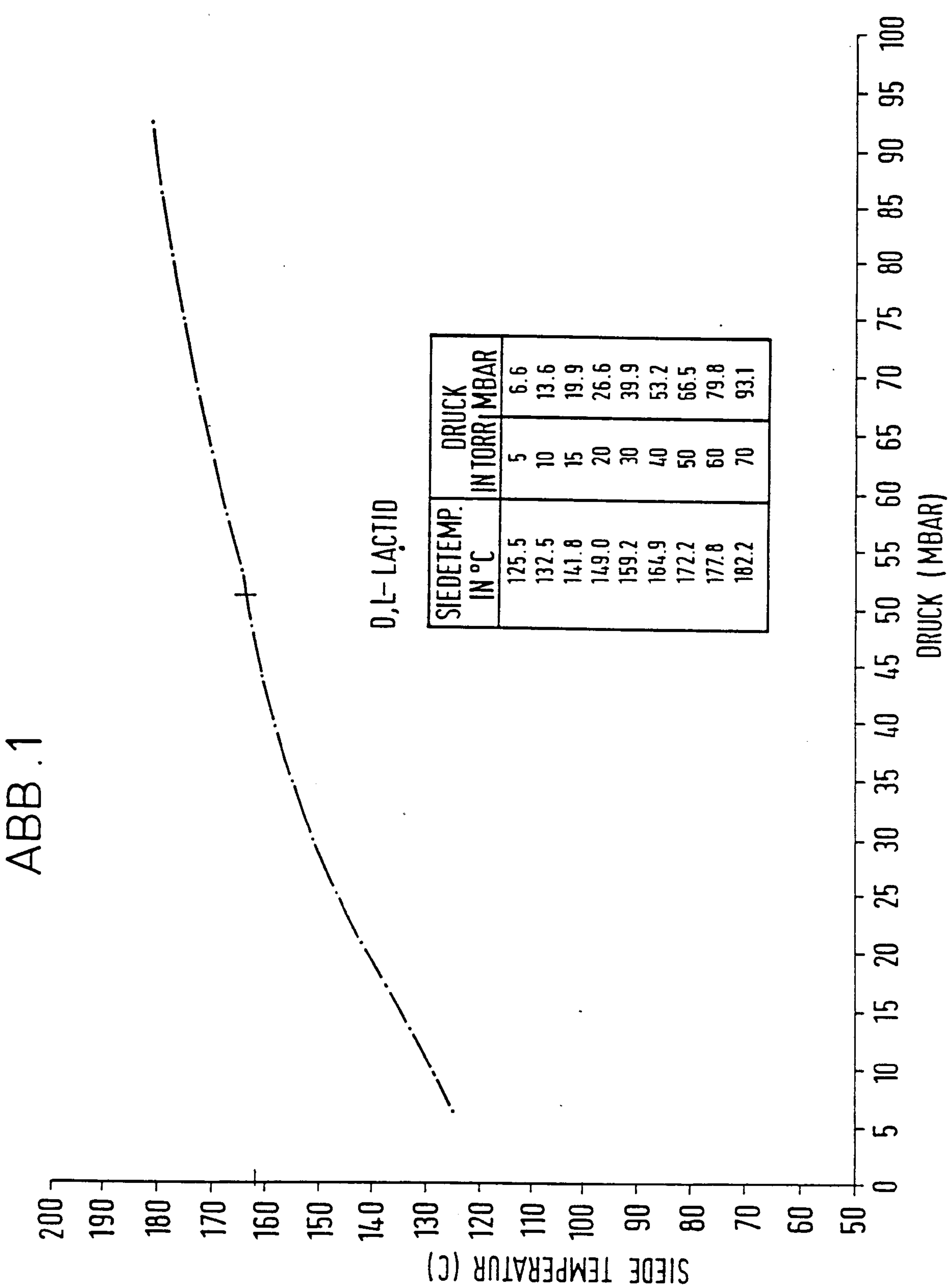


| SIEDETEMP. IN °C | DRUCK IN TORR | DRUCK MBAR |
|---|---|---|
| 125.5 | 5 | 6.6 |
| 132.5 | 10 | 13.6 |
| 141.8 | 15 | 19.9 |
| 149.0 | 20 | 26.6 |
| 159.2 | 30 | 39.9 |
| 164.9 | 40 | 53.2 |
| 172.2 | 50 | 66.5 |
| 177.8 | 60 | 79.8 |
| 182.2 | 70 | 93.1 |

ABB. 2

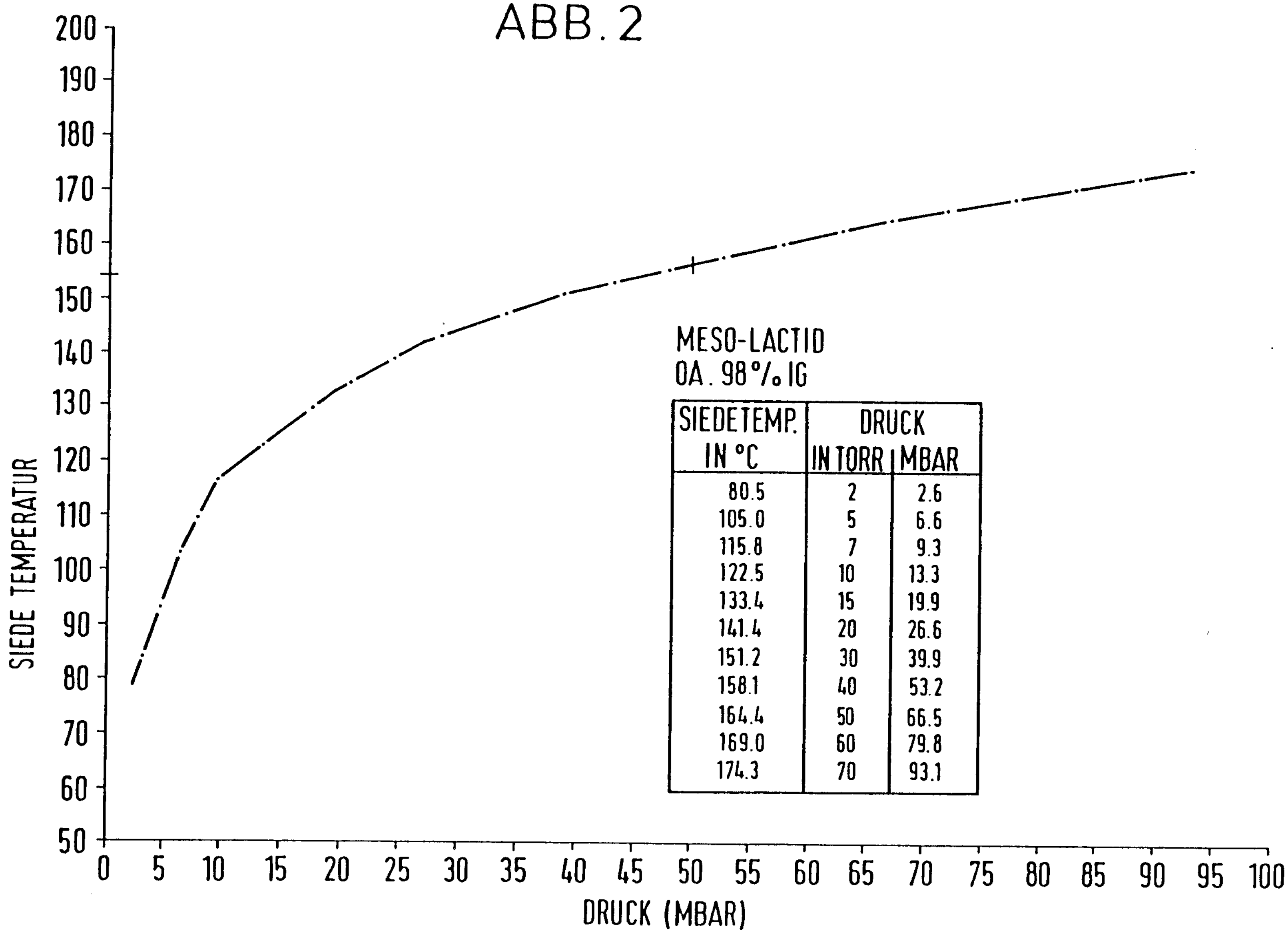


MESO-LACTID
OA. 98 %IG

| SIEDETEMP. IN °C | DRUCK IN TORR | MBAR |
|---|---|---|
| 80.5 | 2 | 2.6 |
| 105.0 | 5 | 6.6 |
| 115.8 | 7 | 9.3 |
| 122.5 | 10 | 13.3 |
| 133.4 | 15 | 19.9 |
| 141.4 | 20 | 26.6 |
| 151.2 | 30 | 39.9 |
| 158.1 | 40 | 53.2 |
| 164.4 | 50 | 66.5 |
| 169.0 | 60 | 79.8 |
| 174.3 | 70 | 93.1 |

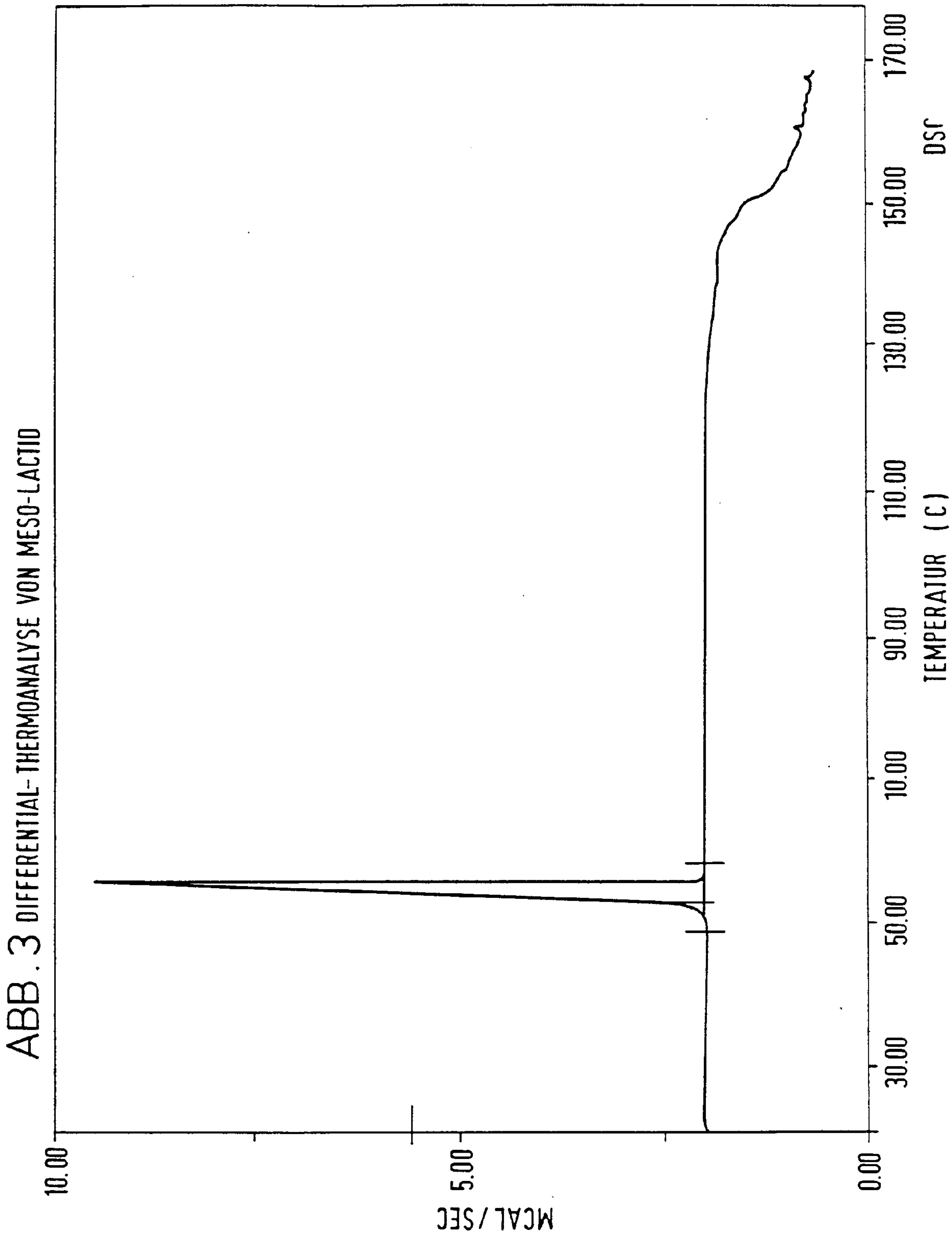


ABB. 3 DIFFERENTIAL-THERMOANALYSE VON MESO-LACTID

ABB. 5 IR SPEKTRUM VON MESO-LACTID IN NUJOL

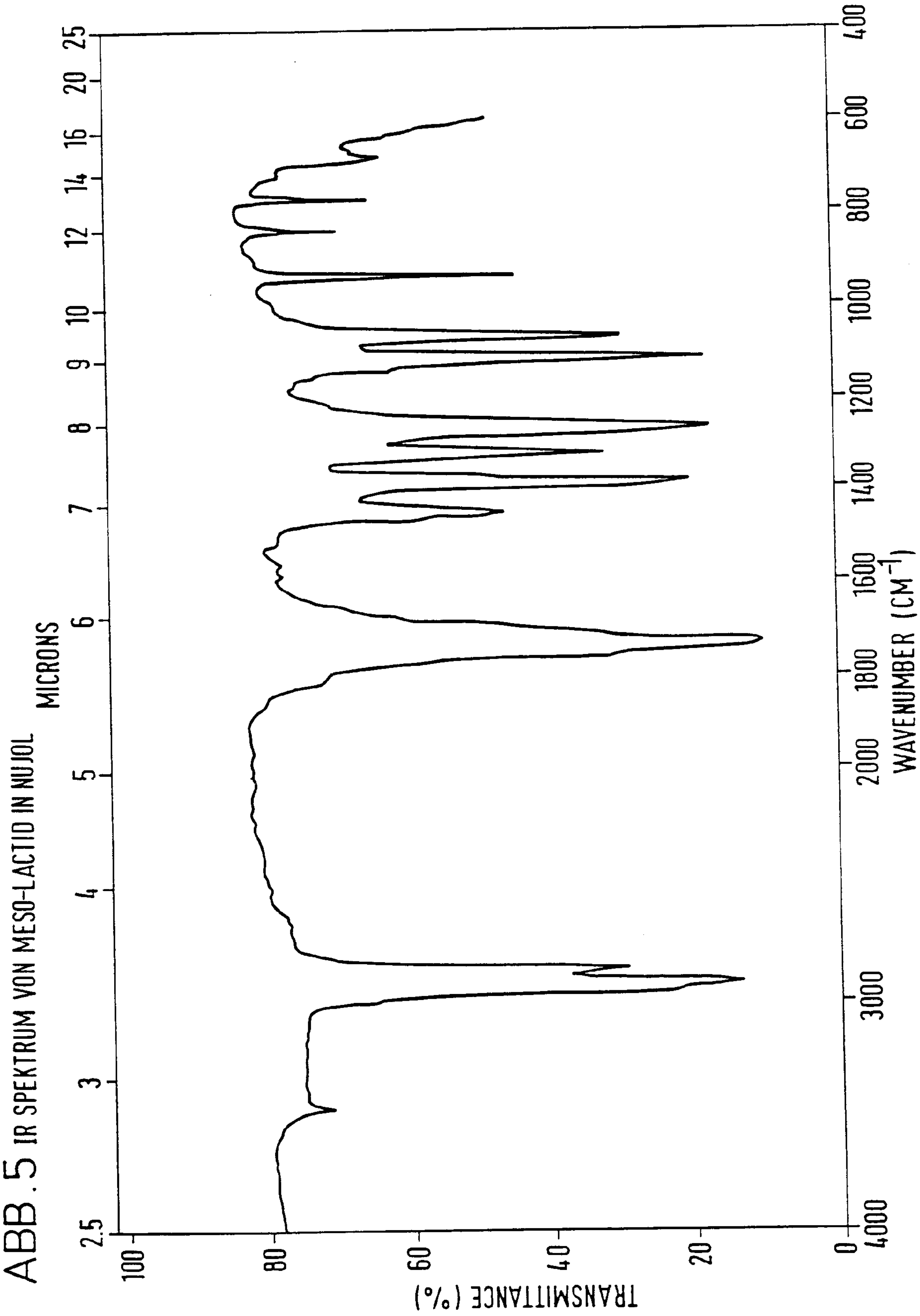

ABB. 4 IR SPEKTRUM VON MESO-LACTID IN KBr

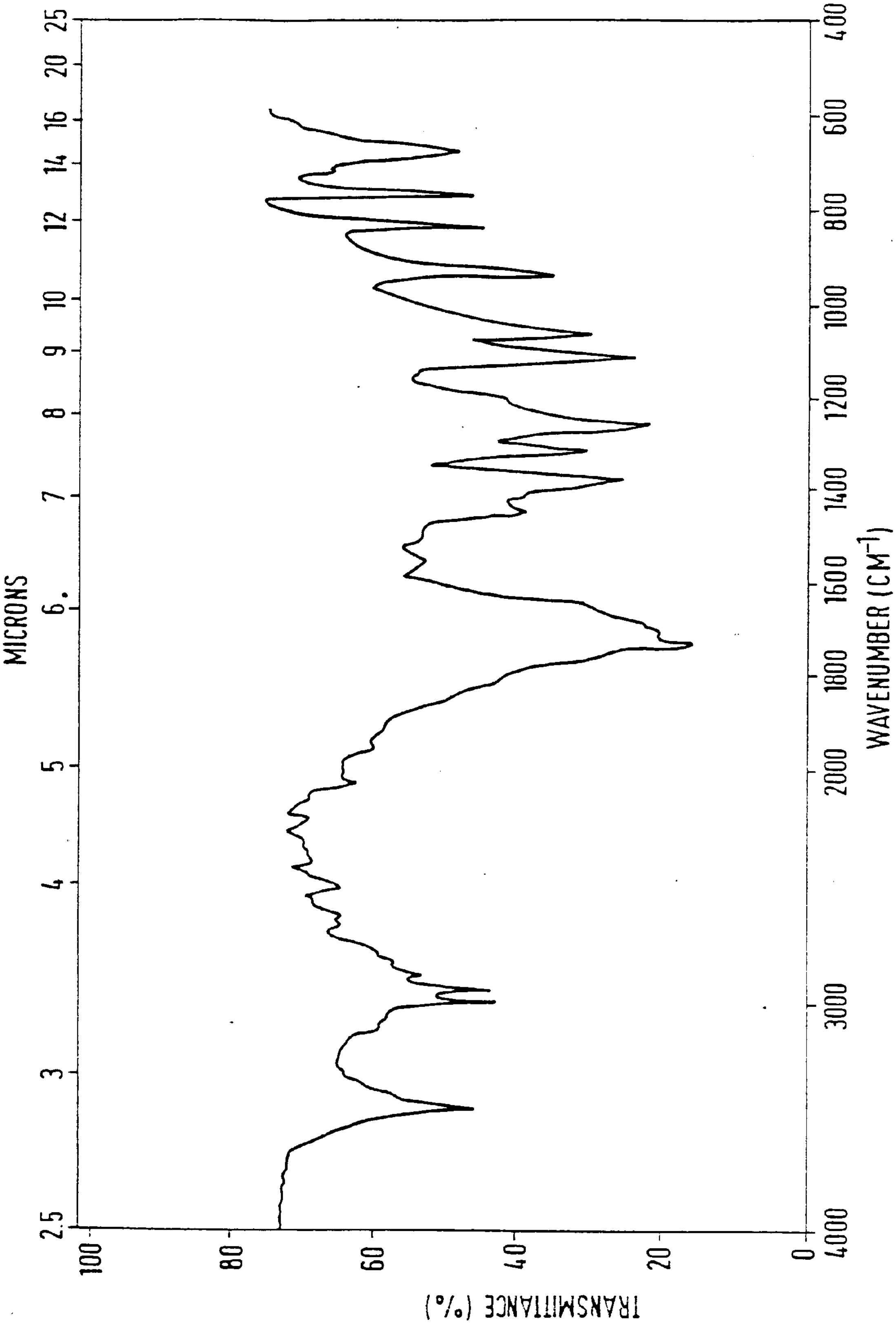

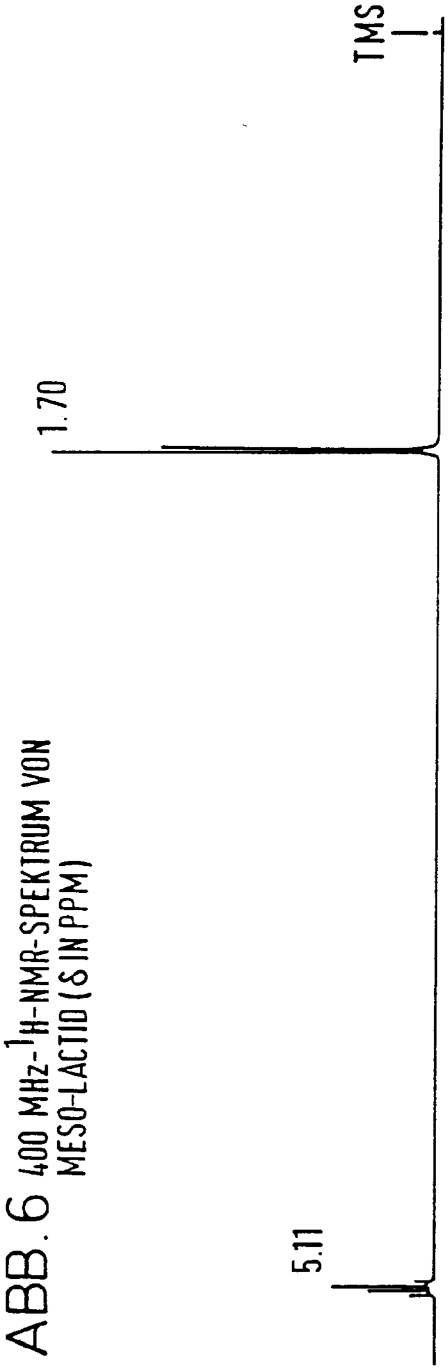


ABB. 6 400 MHz-$^1$H-NMR-SPEKTRUM VON MESO-LACTID (δ IN PPM)

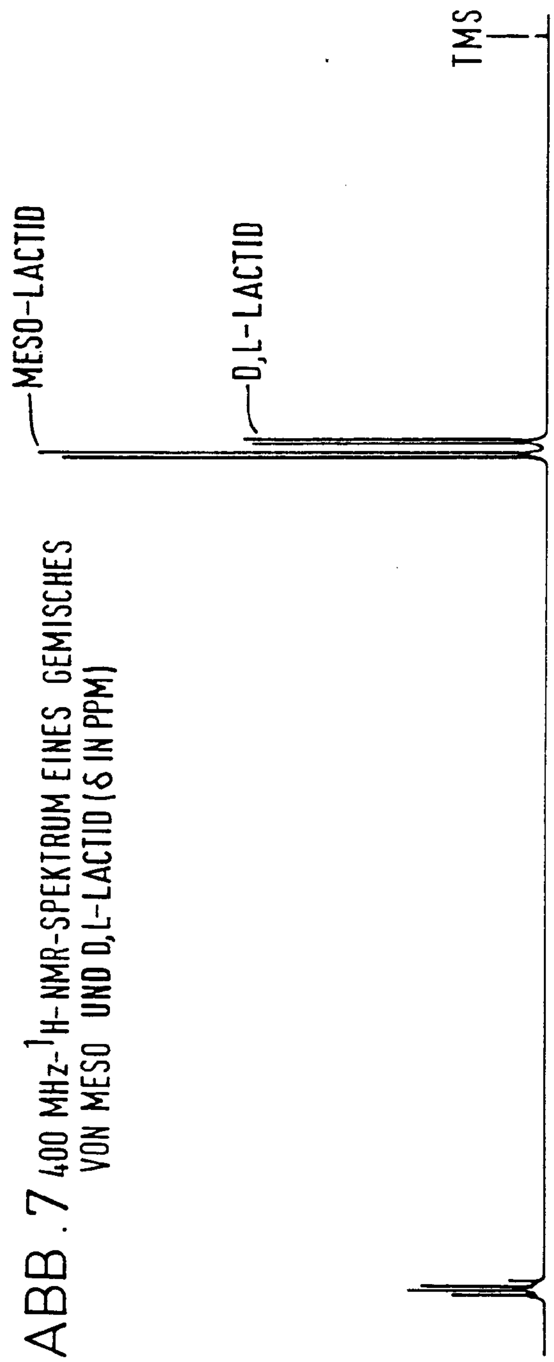


ABB. 7 400 MHz-$^1$H-NMR-SPEKTRUM EINES GEMISCHES VON MESO UND D,L-LACTID (δ IN PPM)